# EUROPEAN PATENT SPECIFICATION

(11) **EP 1 464 345 B1**
(45) Date of publication and mention of the grant of the patent: **30.12.2009**
(21) Application number: 04252022.1
(22) Date of filing: 03.04.2004
(51) Int. Cl.: A61L 24/02, A61L 24/10, A61K 6/097, A61K 6/033, A61L 27/24, A61L 27/46

(54) **Bone growth and adjacent tissue regeneration composition**
Zusammensetzung für die Wiederherstellung von Knochen und umliegendem Gewebe
Composition pour la régénération des os et des tissus adjacents

(30) Priority: 03.04.2003 US 406395; 03.04.2003 US 406681
(43) Date of publication of application: 06.10.2004
(73) Proprietor: Szymaitis, Dennis W., Pittsburgh, Pennsylvania 15205 (US)
(72) Inventor: Szymaitis, Dennis W., Pittsburgh, Pennsylvania 15205 (US)
(74) Representative: Marshall, John Grahame

(56) References cited:
- US-A- 5 550 188
- US-A- 5 702 716
- US-A1- 2002 098 222
- US-A1- 2003 026 770
- US-A1- 2004 062 790

## Description

### FIELD OF INVENTION

This invention relates to an implant material for use in a variety of medical bone growth, bone healing and adjacent tissue regeneration procedures.

### FIELD OF THE INVENTION

The desire to produce an ideal material for facilitation of bone growth and adjacent tissue regeneration are important goals for medicine and dentistry. There are many applications for such a material, including, but not limited to, repair of severe or mn-healing osseous defects (including cranial repair and severe fracture repair), skeletal (especially facial) reconstruction procedures and periodontal surgery aimed in part at regeneration of bone loss from periodontal disease. In the past, autogenic (transplanting the patient's own bone) or allogenic (from bone donated to a 'bone bank') bone grafts have been used. However, procedures using these materials are very expensive and have inherent medical risks. An alternative approach that has gained much attention is to aid bone healing by use of osteogenic or osteoconductive implants. Over the last two decades, calcium phosphate ceramics, such as hydroxyapatite and tricalcium phosphate, and polymers have received the most attention as substitutes for autogenous bone grafts. Calcium phosphate ceramics act through osteoconduction by providing a scaffold for enhanced bone tissue repair and growth. Calcium carbonate is another inorganic material used for bone grafting. It has been reported that a natural coral containing over 98% calcium carbonate and sold under the trademark BioCoral is effective as a bone growth material for periodontal disease. This material is provided as granules 300 microns to 400 microns in diameter. These implants can either be rigid or malleable to fit in the appropriate location and serve the appropriate use in the body. Other insoluble or slightly soluble salts of calcium, bioactive glasses and synthetic, osteoconductive or osteogenic polymer spheres have been proposed for use in this process either by themselves or in conjunction with various additives to form these implants. Another bone graft material is a synthetic bone sold under the trademark Bioplant HJR. This material contains a calcium hydroxide in a co-polymer of polyhydroxyehyl methacrylate and polymethyl-methacrylate. This material is also provided and used in granular form.

It is well known to combine other materials with bone growth materials when used to promote bone growth. Calcium sulfate hemihydrate, also known as medical grade plaster, is often combined with hydroxylapatite to provide initial stabilization and prevent migration to surrounding soft tissues. The calcium sulfate is resorbed by the body within one month leaving a scaffold of hydroxylapatite for bone growth.

Additives to calcium phosphate ceramics have been proposed since the 1960s. The additives that have gained the most attention are natural and synthetic polymeric materials whose functions in the implant include some or all of, holding the bone substitute in place, providing 3-dimensional structure for the implant and acting as an osteoconducter or osteogenic agent. Particularly, collagen has been mentioned in numerous publications and patents as a material that can function suitably in such implants.

While a number of materials and additives have been proposed and some tried for these purposes there still exists the need for the material with the optimal characteristics regarding osteoconductive character, biocompatibility, ability to maintain integrity for the desired time in the body, ease of production, ease of use and cost.

Part of the emphasis for this technology involved uses for bone regeneration material in dental applications, specifically periodontal applications. Periodontal disease occurs when bacteria colonize the sulcus space between the teeth and gingiva. The bacteria cause inflammation. The inflammation destroys the gingival epithelial lining and epithelial attachment to the tooth. The inflammation then progresses down the tooth root toward the apex of the root and destroys periodontal structure and bone. As periodontal disease progresses open pockets develop between the tooth and the gingiva. A dentist can determine the presence and extent of periodontal disease using a probe to measure the depth of pockets between each tooth and gingiva. X-rays can reveal the extent of any bone loss. A common surgical procedure has been widely used to treat bone loss caused by periodontal disease. In this procedure the periodontist uses a scalpel to incise the gingiva and reflects it back to expose the tooth root and bone. Then he removes the irregular shaped bone with hand instruments or rotary instruments, surgically removes granulation tissue and gingiva, cleans the site and places a bone regeneration material into osseous defects that remain in the bone. Guided Tissue Regeneration barriers are placed over bone regeneration material in deeper osseous defects. He then sutures the gingiva around the tooth. Then the gingiva, epithelial attachment, bone, and periodontal ligament between the tooth and bone reform. While this procedure has been effective, incisions in the gingivacause patient discomfort, pain, swelling, gingival recession, sensitive teeth, a long healing time, and increase the possibility of infection. The goal of visualization of the roots, removal of granulation tissue, and excision of damaged gingiva utilizing traditional incision periodontal surgery on a normal compliment of 28 teeth requires a cumulative incision length of 104-117 cm 41 to 46 inches. The extensive cumulative length of surgical incisions wears down the scalpel cutting edge. It is common surgical practice to use and discard between 4 to 10 Bard Parker #15 surgical scalpel blades for each patient. To reposition all of the surgically loosened gingiva requires between 4 to 8 suture packets of 46 cm 18 inch suture. The volume of granulation tissue and gingiva removed is approximately 2 to 5cc. Estimated blood loss varies 2cc to 18cc per patient. The post-surgical period requires strong analgesics to maintain pain relief. At the 2 week suture removal appointment, the gingival incisions are only about 50% healed, and require 2 to 4 additional weeks for final healing. The period of painful, sensitive, and bleeding gingiva lasts 3 to 4 weeks after the surgery. After this struggle to gain periodontal health, the patient now faces gingival recession with exposed sensitive roots resulting from granulation and gingiva removal. Cosmetic difficulties result from the loss of the interdental gingiva that creates dark spaces between the teeth. The exposure of the margins of facings and crowns often requires replacement to correct cosmetic deficiencies. Consequently, there is also a need for a procedure for reversing bone loss and periodontal structure damage caused by periodontal disease. There is also a need for a regeneration material that could be used without incisions to regenerate bone and periodontal structure lost to periodontal disease. Periodontal therapy without incisions, eliminates discomfort, pain, swelling, gingival recession, sensitive teeth, greatly shortens healing time, and greatly decreases potential for infections. One problem associated with the use of the bone growth materials described above is that the particle sizes require incisions in the gingiva to apply the material. However, in addition to the problems mentioned above, surgical procedures always require more healing time than procedures that do not require incisions. However, the art has not developed a procedure for treating bone loss from periodontal disease that does not require incisions in the gingiva. Consequently, there is a need for a bone growth material that can be placed adjacent to a degenerated alveolar bone to promote bone growth without requiring incisions in the gingiva. There is also a need for a procedure to place such a material to reverse periodontal disease without incisions in a patient's gingiva. Such a procedure and material should not merely grow bone. Rather, they should result in reforming of the epithelial attachment and encourage periodontal structure regeneration.

Any composition that is injected into the periodontal pocket or otherwise placed on a bone must not substantially migrate out of the pocket or away from the bone after injection or placement. While the injectable gel-type bone growth compositions disclosed in the prior art could be injected into the periodontal pocket, they migrate out of the pocket before they have much effect as a result of normal rebound or retraction of the distended pocket and movement of the mouth.

### REVIEW OF RELEVANT LITERATURE

The literature regarding novel bone re-growth and osteoinductive compositions can be broken down into three distinct classes: (1) use of naturally or man-made insoluble or slightly soluble calcium-containing solids with or without other additives, (2) use of man-made materials other than calcium-containing solids (e.g., bioactive glass, organic polymers) in osteoinductive compositions and (3) use of natural or synthetic bone growth factors which can be used to speed bone re-growth (see, for example, U.S. Patent No. 6,617,307 Nishimura, et al and references therein).

Literature containing information on using bone materials for bone graft and bone re-growth applications extends at least back to 1965. Many patents mention the use of hydroxyapatite (also known as hydroxylapatite or HA) (see below) as well as other bone materials such as highly processed bone (U.S. Patent No. 5,501,706), calcium phosphate cements (U.S. Patent Nos. 5,697,981; 5,783,217; 5,683,461; 5,605,713; 5,522,893), physiological serums containing hydroxyapatite (U.S. Patent No. 5,591,232), sintered apatite bodies and composites (U.S. No. Patent 4,503,157).

In many cases it is advantageous for bone growth materials, especially calcium phosphate ceramics, to be admixed with a matrix-forming material to create a uniform material to help the bone materials stay associated. One such material which has been widely tested is collagen, which may also function to help facilitate bone re-growth. The use of mixtures of collagen protein and bone minerals has been suggested for some time (see, for example, Levy, P., et al J. Periodontal (1981), 52:303-6). Many patents and patent applications have mentioned collagen and bone material.calcium phosphate ceramics (including hydroxyapatite). Examples include U.S. Patent Nos. 6,617,307 (Nishimura, et al), 6,599,516 (Knaack), 6,589,590 (Czernuszka, et al), 6,583,248 (Bowen), 6,506,217 (Arnett), 6,387,414 (Akashi, et al), 6,201,039 (Brown, et al), 6,187,047 (Kwan, et al), 5,990,381 (Nishihara), 5,851,670 (Mitoh, et al), 5,776,193 (Kwan, et al), 5,739,286 (Silver, et al), U.S. Patent Nos. 5,707,962 (Chen, et al); 5,425,770 (Piez, et al, divisional of 4,795,467), 5,352,715 (Wallace, et al), 5,338,772 (Bauer, et al), 5,292,253 (Levy), 5,178,845 (Constantz, et al), 4,795,467 (Piez, et al), 4,780,450 (Sauk, et al), 4,472,840 (Jefferies), 4,394,370 (Jefferies), 4,314,380 (Miyata, et al), PCT Applications WO/15653 (Ammann, et al) and 95/08304, British Patent Specification 1,271,763, Japanese Application J58/058041 and European Patent Application, Publication No. 030583. Collagen can exist in two forms cross-linked and not cross-linked. To the extent that the literature has described the collagen used in bone growth compositions, that collagen has been the cross-linked form. However, I have discovered that collagen which is not cross-linked functions differently from cross-linked collagen in a bone growth composition.

A number of commercial products have even been described that contain collagen and calcium phosphate materials. These include Collegraft® (Zimmer, Inc., Warsaw, Ind.) and Biostite™ (Vebas Dental Co, Italy).

Various methods of preparation, the addition of various additives, kit formation and even methods of sterilizing the final product have been described for calcium phosphates (including hydroxypatite, sometimes called HA). U.S. Patent No. 6,506,217 discloses a composition of AVITENE microfibrillar collagen, hydroxyapatite and a fluid, which can be saline that is cured after mixing and molding to produce a dessicated, shaped, hard implant. Curing is accomplished by a heat lamp, a heated oven or a dehydration unit containing a dessicant. The implant is placed in the body using standard surgical procedures. U.S. Patent No. 6,583,248 teaches HA and/or collagen residing in the interstitial spaces in cyclodextrin polymers. U.S. Patent No. 6,599,516 teaches precipitating calcium phosphate on the surface of a collagen membrane by allowing a calcium solution and a phosphate solution on different sides of the membrane to diffuse together. U.S. Patent No. 6,599,516 teaches a malleable implant using HA and collagen where the collagen is said to dissolve before the HA, allowing access for in-growth of new bone and also allows for addition of other materials. This implant may be hardened by curing step by crosslinking reactions or by hydration in the case of some inorganic materials. A kit is mentioned with one container containing powdered bone growth material. The material may be injected as a paste preferably through an 18 or 16 gauge syringe. In the case of the paste the HA would be injected first and the collagen injected afterwards into the site of desired bone growth. U.S. Patent No. 6,187,047 teaches a ternary composition with insoluble collagen, HA, and a soluble binder such as soluble collagen. The integrity of the HA/collagen composition must be maintained for at least 3 days and porosity must be maintained for 7-10 days. A potential embodiment uses cross-linking agents to help "cure" the composition to help maintain integrity and porosity. Finally, this patent teaches the use of radiation, chemical and autoclaving methods of terminal sterilization of the final product. U.S. Patent No. 6,201,039 teaches a kit for making HA/collagen composition using two vials, one containing HA powder and one containing collagen in a liquid, including non-aqueous, non-polar liquids. U.S. Patent No. 6,323,416 teaches the substitution of some of the ions in HA with boron or silicon atoms although it does not specifically mention collagen in the claims. U.S. Patent No. 5,990,381 teaches the use of shark-derived collagen with HA due to the extraordinary healing powers of shark collagen. U.S. Patent No. 5,851,670 teaches HA and collagen and using the composition for drug delivery but also focuses on collagen and other biopolymers being coated by HA a mechanism of action. U.S. Patent No. 5,776,193 teaches of an HA/collagen composition that is given three-dimensional structure by use of cross-linking agents designed to make a rigid osteoinductive solid matrix to facilitate bone growth. U.S. Patent No. 5,425,770teaches mixing collagen, HA and a liquid in a mold and drying in that mold. U.S. Patent No. 5,352,715 teaches HA and collagen with a variety of fluids including polyethyleneglycols (PEGs), hyalurionic acid and poly (hydroxyethyl methactylate) which makes the composition a gel. This patent also teaches a malleable, syringe-injectable formulation that may contain other bioactive compounds as additives. U.S. Patent No. 5,338,772 teaches for HA/collagen compositions that contain at least 50% by weight HA, that microwave heating can be used to formulate the composition as long as essentially dry collagen and HA are mixed together before microwaving, which this patent defines as the only reliable method (including microwave lamps and conventional ovens) that can be used. U.S. Patent No. 5,178,845 teaches that the collagen can be added to the HA before the large HA particles are ground to give various viscosities that can be further modified by the addition of pure water to the formulation. U.S. Patent No. 4,795,467 teaches heating the HA/collagen essentially dry mixture between 60 and 120 degrees centigrade for 4 to 168 hours. The patent also teaches that optimum results are obtained by heating the mixture for long times of 1-10 days and that improvement could be obtained by increased cross-linking of the collagen. U. S. Patent No. 4,780,450 teaches HA, collagen and phosphoryn calcium slats slurried in water as a potential bone growth material. U.S. Patent No. 5,707,962 teaches that a porous, solid polymer matrix (e.g., collagen) can be treated with various additives such as calcium phosphates (including hydroxyapatite), bone morphogenic proteins, antibiotics and other additives. U.S. Patent No. 5,292,253 describes a procedure in which missing bone is filledwith a mass of calcium phosphate or hydroxyapatite and covered with a gel containing a collagen, fibrin or gelatin and a dye. The gel is then exposed to potentially harmful laser radiation to weld the calcium containing material to the bone.

Despite the many proposed compositions containing bone growth materials and collagen, none have provided improved or more rapid healing over other bone growth compositions. Many suffer problems caused by migration of the bone growth material away from the application site. Most must be applied using invasive surgery.

There is a need for a bone growth material that will remain in place and encourage tissue growth. This material should be able to be applied to the site of bone loss without invasive surgery. Preferably the composition should be able to pass through a syringe and injected into a periodontal pocket or other bone growth site.

### SUMMARY OF INVENTION

The present invention provides a composition to produce a malleable, ductile material that provides a successful support for growth of new bone and adjacent soft tissue in a variety of medical procedures and may also function in drug delivery. The invention provides a composition according to claim 1. The composition contains one or more bone growth materials, free collagen, a small amount of saline or other fluid, and, optionally, other additives to modify the consistency, ionic nature, antibacterial nature, color, texture, smell, osteogenic capacity, resorptive time (if any) and, if necessary, the rigidity of the implant. These compositions can be formulated by including addition of energy (e.g., heating) using a variety of sources.

In a preferred method of manufacture the composition is heated to dissociate the collagen fibers and allow at least some of the bone growth materials and any additives to be captured between adjacent fibers without denaturing any proteins in the collagen. Dissociated collagen provides initial implant stabilization similar in function to calcium sulfate. Because the bone growth materials are captured in the collagen they tend to remain in place and not migrate from the site. Collagen encourages tissue growth, particularly growth of blood vessels through the composition. Collagen provides additional benefits by attracting healing cells and is a natural biologic material tolerated by patients. Consequently, bone growth and healing are faster than occurs using bone growth compositions known in the prior art. These inherent properties of free collagen provide for an active role in healing, unlike other stabilization materials that only provide stabilization.

Other objects and advantages will become apparent from a description of certain present preferred embodiments of the composition and methods of using same which are shown in the Figures.

### BRIEF DESCRIPTION OF THE DRAWINGS

Figure 1 is a front view of a healthy tooth.
Figure 2 is a front view of a tooth in a patient experiencing periodontal disease.
Figure 3 is a front view of a tooth in a patient having advanced periodontal disease and bone loss.
Figure 4 is a front view of the diseased tooth of Figure 3 being treated in accordance with the present invention.
Figure 5 is a front view of the diseased tooth of Figure 3 immediately after completion of a treatment in accordance with one embodiment of the present invention.
Figure 6 is a top plan view of a preferred embodiment of a periodontal kit with most of the cover having been cut away.
Figure 7 is a perspective view of a preferred embodiment of a preferred gauze placement tool in the kit of Figure 6.
Figure 8 is a perspective view of a present preferred dental bur in the kit of Figure 6.

### DESCRIPTION OF THE PREFERRED EMBODIMENTS

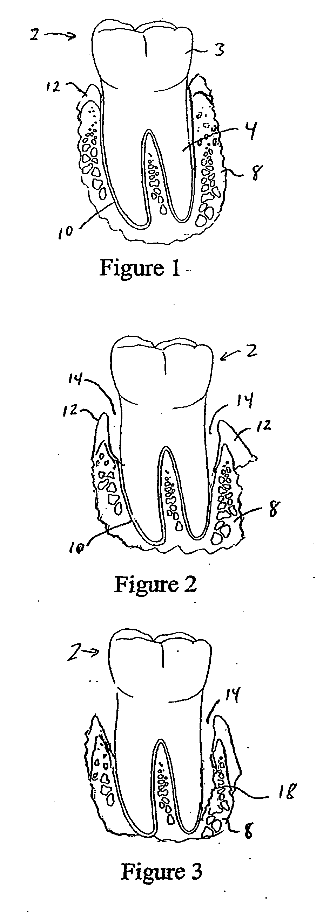

The composition of the present invention is particularly useful for treatment of periodontal disease. As can be seen in Figure 1 a tooth 2 has a crown 3 and root 4. The alveolar bone 8 surrounds the root 4. There is a periodontal ligament 10 around much of the root 4 and gingiva 12 surrounds the upper part of the root and the base of the crown 3. There is a pocket 14 between the gingiva 12 and the tooth 2. In a healthy tooth shown in Figure 1 this pocket 14 is very shallow, typically one to three millimeters. Bacteria colonize the pocket. The bacteria cause the pocket 14 to deepen as shown in Figure 2. As periodontal disease progresses the bacteria cause inflammation that destroys the ligament and bone creating depressions 18 in the bone shown in Figure 3.

The conventional way of treating advanced periodontal disease is to create incisions to reflect the gingiva 12, remove the damaged bone with hand instruments or sand area 18 with rotary burs, remove all debris from the sanding, place a bone growth material adjacent to the sanded bone surfaces and suture the gingiva. The standard procedure for deeper osseous defects is to place expensive, and very difficult to manipulate, Guided Tissue Regeneration barriers over the bone regeneration material prior to suturing. A more preferable method would be to provide a method and material that does not involve incisions in the gingiva. Instead a bone growth material finely ground and mixed with free collagen particles is injected into the pocket through a syringe. Prior abrading of the root surface and inner surface of the gingiva is conducted first with a dental bur. Improved bone and periodontal structure regeneration will be seen if the abraded particles are not taken out of the pocket. Tooth dentin is a natural source of bone morphogenic protein and, by not rinsing, this is utilized as a source to promote bone generation. This failure to rinse is contrary to present dental practice. Standard periodontal practice is to completely clean the pocket of all abraded bone and to remove all granulation tissue To abrade the root and gingival surfaces the dental bur is simply placed into the pocket, without cutting the gums. After abrading, a specially designed sponge is placed into the 1-1.5mm created space to both distend the pocket and create hemostasis.

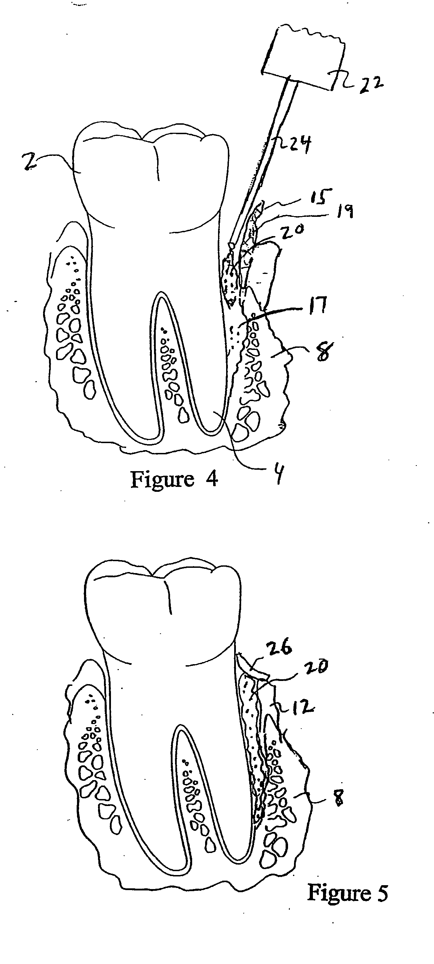

Each sponge is a gauze material of the type used in dental and surgical procedures which has been cut into strips. The terms sponge and gauze are used interchangeably herein to refer to such material which is sometimes also called surgical sponge or surgical gauze. It is preferred to provide three sizes of gauze strips of 5 mm, 7.5 mm and 10 mm wide and 10 cm in length. Many surgical sponges including Nu Gauze surgical sponges are made from materials that tear easily with slight pulling force. If this type of sponge or gauze is used, a piece oftorn gauze may be left in the pocket when the sponge is removed. For that reason this type of surgical sponge should not be used. Nevertheless, I have found that such sponges could be modified to contain reinforcing fibers that prevent tearing. The sponge 15 in Figure 4 is shown to have such reinforcing fibers 19. Further, the sponge or gauze can have distinct markings on the ends to determine if the entire gauze strip has been totally removed and that no inadvertent tearing of the gauze that would allow a torn piece to remain in the pocket. The gauze can carry and place astringents, antibacterial materials, antibiotics, collagen, bone growth substances, or any type of root conditioning materials. Sufficient time is allowed for hemostasis, distention, and the placed materials to function as designed, then the gauze 15 is removed. As shown in Figure 4, after abrading a periodontal structural regeneration composition 20 is injected into the pocket 14 through a syringe 22 and needle 24. This composition contains fine particles of bone growth material, free collagen particles, a small amount of saline or other fluid and optional additives which may be either soluble or insoluble particles, all preferably sized to be less than 1 mm in diameter. Free collagen particles are not cross-linked and are available to speed healing. The collagen may be small fibrils or a gel containing fibrillar collagen. A small amount of sterile saline or other liquid may be added.

Bone growth materials may be chosen from commercial sources, could be synthesized from a variety of starting materials or harvested with or without processing from animal or human bone. Commercially available bone growth materials include, but are not limited to, bioactive glass such as is sold under the trademark Biogran®, calcium phosphate derivatives including those sold under the trademarks Osteogen®, Bio-Oss, Laddec® , Norian Skeletal Repair System, Osteograf®, Interpore 200®, Collegraft Zimmer, BioSource®, Capset®, Cerasorb® and Dembone® and chemically modified calcium phosphates such as that sold under the tradmark Biostite™. Other bone growth materials such as those sold under the names Bioplant HTR®, BOP, α-BSM® (one version sold under the name of Biobon® or embarc®) can be used. In addition to commercially available bone growth materials described above, other insoluble or slightly soluble calcium-containing materials including synthetic hydroxyapatite and other calcium phosphate cements may be synthesized as described in the peer reviewed literature and the patent literature. Examples of materials and syntheses are given in U.S. Patent Nos. 6,616,742; 6,521,264 (Lacourt, et al); 6,506,217, 6,599,516; 6,323,146; 6,201,039; 5,690,908; 5,609,850; 5,595,724; 5,520,904; 5,468,465; 5,344,640 and 5,407,659 and in Artzi, et al Int. J. Periodontics Restorative Dent. 2003, Aug:23(4):381-9 and Gabrielli, et al Braz. Dent. J. 2001; 12(1):9-15 and references therein. Anhydrous calcium sulfate can also be used as a bone growth material. The described materials and synthetic methods are incorporated herein by reference. It should be noted that these descriptions include reference to substitution of calcium, phosphate or hydroxide ions in these preparations for other ions or combinations of ions to modify properties of the resulting apatite particles including, but not limited to, solubility, hardness and color.

Matrix forming materials in addition to free collagen may be added to the composition and may or may not aid in bone formation themselves. They may be materials that, optionally, aid in changing the viscosity of the composition by increasing or decreasing the viscosity of the composition to maximize ease of use in the procedure and to minimize the loss of material from the site of application, such loss lessening the effectiveness of the treatment using the composition. Matrix forming agents that could be used in this invention either alone or in combination with each other would be other forms of collagen under 1 mm (see, for example, commercially available forms of collagen such as, but not limited to Kollagen™ ,Avitene™, Collastat™ or Zyderm® or collagen as described in U.S. Patent Nos. 4,780,450 and 4,795,467 and references within or combinations of one or more forms of collagen), agents described in section 11.3 of the Standard Handbook of Biomedical Engineering and Design, including water soluble polymers, like polyethylene glycols, polyvinyl pyrrolidones, hyaluronic acid, dextran and starch, gelling polymers such as poloxamers, alginates, gelatins and fibrin, hydrogels such as PHEMA, chitosans, collagen and various cellulose materials including oxidized cellulose. Gelatin (WO 98/40113 (Wironen, et al)), dextrans (WO 00/15274), chitosan hydrogels (U.S. Patent No. 6,124,273) have been described to be used in conjunction with calcium phosphate cements. Another possible type of matrix-forming agent, hydrocolloids, are hydrophilic polymers, of vegetable, animal, microbial or synthetic origin, that generally contain many hydroxyl groups and may be poly-electrolytes. These materials can control properties such as viscosity (including thickening and gelling) and water binding. Aside from the hydrogels described above, other hydrocolloids such as but not limited to, carrageenan, carboxymethylcellulose and xanthan gum may be useful alone or in conjunction with other matrix forming agents. Other matrix forming materials, such as temperature-sensitive thickening agents which melt above body temperature but solidify at or slightly above body temperature, would be very useful as matrix forming agents in the present invention. Examples include colloids that melt above 38 degrees centigrade (100 degrees Fahrenheit) and polyethylene glycols as described in U.S. Patent No. 5,352,715 and biodegradable polymers that are thermoplastic or thermosetting (U.S. Patent No. 5,324,519 (Dunn, et al)) or in situ solidfying (U.S. Patent No.RE 37,950 (Dunn et al.)). Other types of matrix-forming materials include synthetic peptides (PepGen P-15 by CeraMed Dental), poly (L-lactide) (Rosen and Reynolds J. Periodontol. 1999 May; 70(5): 554-61 and U.S. Patent No. 5,851,670 (Mitoh, et al)), poly (L-lactide)-epsilon-caprolactone copolymer (Senkoylu et al Int. J. Artif Organs 2002 Dec; 25(12):1174-9), poly(propylenefumarate) (Lewandrowski, et al J. Biomater Sci. Polym Ed. 2000; 11(8):879-89), biodegradable terephthalate polymers (U.S. Patent No. 6,600,010), natural polymers succinic acid/carboxymethyl chitin (Yokoyama, et al J. biomed Mater Res 2003 Mar 1: 64(A(3):491-501), other biopolymers (references in U.S. Patent No. 5,338,772 (Bauer, et al) and polyphosphates (U.S. Patent Nos, 6,537,589 (Chae, et al) and 6,406,711 (Lee et al)).

The fluid can be chosen from normal saline, water for injection (i.e., sterile water), inorganic salt solutions or combinations thereof. The fluid can also contain small amounts (under 10%) of water-miscible organic solvents that will not precipitate any salts in the fluid nor interfere with the favorable properties of the composition.

Additives for use in this composition may include wound healing compositions (U.S. Patent No. 6,503,539 and references therein) applied near or at the top of the tooth-gum interface, microspheres containing additives, for example, antibiotics (U.S. Patent No. 6,193,994), bone morphogenic protein or, more generally, the transforming growth factor (TGF) beta family of compounds (Wozney JM et al, Science, 1988; 242:1528 and U.S. Patent 6,617,307 and references therein), polymers that may or may not be later crosslinked, cross-linking agents specific to those polymers (e.g., see description in U.S. Patent Nos. 6,187,047;6,509,031; 4,394,370; 4,472,840 and 4,795,467) to provide three dimensional structure to the composition, phosphoryn calcium salt (U.S. No. Patent 4,780,450), cells of living tissue such as osteoclasts, chondocytes, bone resorption regulators (e.g., OP-1, parathyroid hormone and others mentioned in U.S. Patent No. 6,599,516) and other additives as described in WO 98/16268. Inclusion of drugs, topical pain relievers, antimicrobial agents, inorganic salts, simple sugars, thickening oligosaccharides, preservatives, lubricating agents, small amounts of acid, anti-inflammatory agents, texture-inducing agents, coloring agents and agents to generate good smell and taste of the composition also may be included as needed.

Methods of manufacture for the bone growth and adjacent tissue regeneration composition useable for this invention include, but are not limited to:
(a) hand or mechanically mixing the materials used to manufacture the composition including grinding the bone growth material separately or together with the free collagen and/or the fluid and/or additives before formulation;
(b) mixing the bone re-growth material, the matrix-forming material and the fluid, formulating and, at a later time, adding other materials (additives) as desired, and
(c) pre-treating the bone growth material to insure one or all of: a particle size under 400 microns, increased or decreased porosity, and change in surface properties to allow better interaction with the matrix forming materials.

After mixing of the bone growth material, free collagen and fluid, and, optionally, some additives, I prefer to apply energy in the form of heating using a water or oil bath, microwave energy, radiant heat (e.g., a heat lamp), or sonication (using a conventional sonic bath or a more powerful sonic probe such as is disclosed in U.S. Patent No. 5,690,908). Heating must be such that the collagen fibers dissociate and allow bone growth material and other additives to be captured between adjacent collagen fibers. But the heating must not denature the proteins in the collagen. The composition is then cooled and can be stored. Optionally, this heating and cooling cycle can be repeated up to five times to either slightly modify the characteristics of the composition or to re-use a composition. While these are preferred methods of manufacture any method that does not cause the composition to become unusable in the medical procedures described below is acceptable.

Examples of medical procedures and uses that may benefit from use of the composition here disclosed, but are not limited to, orthopedic surgery bone augmentation (including helping to heal difficult fractures, stopping resorption of bone and augmenting bone grafts) maxillofacial reconstructive surgery (when used as a rigid solid or in conjunction with a rigid matrix), and procedures used to combat the results of periodontal disease. The composition may be used for drug delivery or other pharmaceuticals. The composition is useful in many animals as well as in humans.

When used to treat periodontal disease, the composition may also contain a root treatment material such as a bone morphogenic protein or a dental matrix derivative available from the Swedish company Biora under the trademark EMDOGAIN. Furthermore, a growth factor, nutrient factor, drug, anti-inflammatory agent, anti-bacterial agent, antibiotic, calcium containing compound or combination of these materials may be included in the composition.

Referring again to Figure 4, sponge 15 should be sized to fit within the periodontal pocket and be able to place proper pressure on the bleeding gingiva to stop the bleeding. Therefore, a compressible sponge should be used to allow compaction enabling the sponge to fit the irregular pocket. A modified Nu Gauze surgical sponge cut into small strips that are 5mm to 10mm wide and 10 cm long is a suitable compressible sponge.

I prefer to inject the composition using an 18 gauge needle 24 because larger needles do not permit access to the small pockets found around the smaller anterior teeth. A 16 gauge needle could be used for treating molars suffering periodontal disease. The particles in the periodontal structure regeneration composition 20 must be small enough to pass through the selected needle without clogging. Because the particles are so small the periodontal structure regeneration composition 20 looks like a gel as it exits the needle. This composition mixes with abraded root particles 17 in the pocket. Sufficient periodontal structure regeneration composition 20 is injected to fill the pocket as shown in Figures 4 and 5. The gingiva 12 then rebounds from the distension and moves back toward the tooth. Because the composition is injected into the periodontal pocket through a needle the viscosity has to be sufficient to pass through the needle but viscous enough to remain in the pocket. Some composition is forced from the pocket when the gingiva rebounds or returns to its normal position. But, most of the periodontal structure regeneration composition remains in the pocket. However, the viscosity of the composition that allows easy passage through the needle enables further migration from the periodontal pocket during normal movement of the mouth. In one embodiment, the composition is formulated to thicken after placement. In another embodiment, the composition may not thicken but the pocket is sealed. After a less thick composition that may migrate easily from the pocket is injected I prefer to apply an adhesive 26 over the pocket to eliminate any pain or discomfort, to stop any bleeding, to keep the regeneration material in place, and to prevent foreign material from entering the pocket and dislodging the regeneration material. However, it has been found that enhanced (as compared to no composition addition) healing does occur even where the pocket is not sealed and composition remains in the periodontal pocket. A preferred adhesive is butyl cyanoacrylate because this material cures with water. Another suitable water curable adhesive is 2-Octyl Cyanoacrylate sold by Johnson & Johnson under the trademark DERMABOND. One could also use an adhesive that cures when exposed to light. Dentistry now uses polymers for filling cavities that cure when exposed to blue light. There are also adhesives that cure when exposed to ultraviolet light that could be used. However, far too many eye injuries caused by ultraviolet light have resulted in a general reluctance to use ultraviolet light in dentistry. There are adhesives that cure upon exposure to visible light including blue light which should be useful in this procedure. Other water curable adhesives, auto-curing adhesives, heat cured adhesives and reactive-component cure adhesives may also be acceptable.

Over time the periodontal regeneration composition causes both gingival growth, bone growth, and formation of a new epithelial attachment. Depending upon the extent of the disease, the teeth and bone will return to their healthy status shown in Figure 1 in about 3 to 12 weeks.

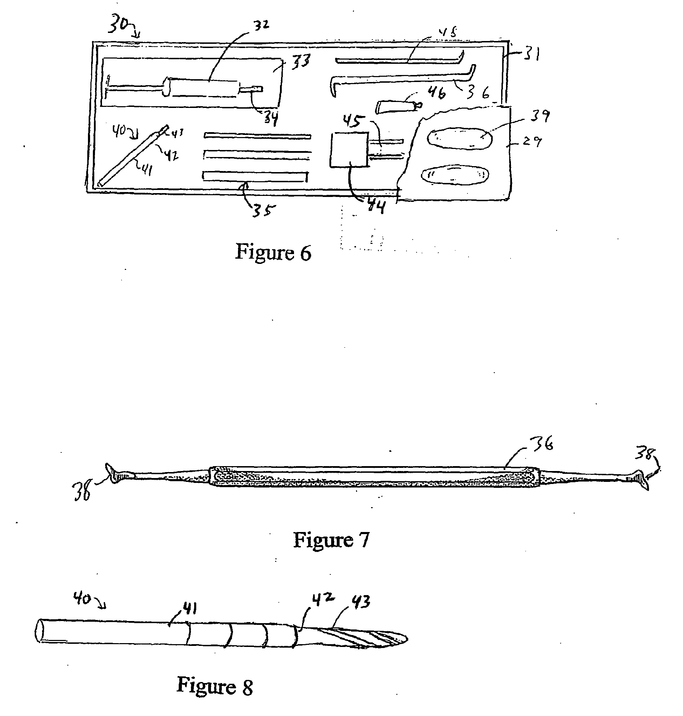

Because the present periodontal structure regeneration compound can be placed in the periodontal pocket to initiate bone growth without surgery, this composition and method for injecting the composition could be used by any dentist. To make the composition and procedure easier to use it is preferred to provide periodontal kits such as are illustrated but not to scale in Figure 6. Smaller items are enlarged and larger items are reduced in a size so that all items could be clearly seen. Each kit 30 is a container 31 with a cover 29 in which there are one or more syringes 32 with 18 gauge needles 34 in a sterile package 33. The syringes are prefilled with from .1 ml to 1.0 ml of periodontal structure regeneration compound. If the thickener is of the type that must be mixed with a catalyst to initiate hardening one could use the type of syringe disclosed in United States Patent No. 4,743,229. In this type of syringe the catalyst is in one syringe that is separate from the reminder of the composition that is in a second syringe. The two syringes are connected by a channel that enables the components to be mixed by passage through the channel. The kit also contains a supply of surgical sponges 35 that have been sized into strips to fit within the periodontal pocket. The kit preferably contains three widths of sponges all the same length. The sponges may contain an antibacterial chemical such as peroxide, chlorhexadine, iodine and triiodomethane or other antibacterial chemical. The sponge may also contain a root treatment material, such as a citric acid, fluoride, or EDTA. The sponge may also be impregnated with at least one of epinephrine, alum, aluminum sulfate, aluminum, potassium sulfate, aluminum chloride, oxyguinol sulfate.

It is also preferred to provide a sponge placement instrument 36 shown in Figure 7. The ends of the sponge placement instrument are shaped to have curved surfaces 38 that conform to the contours of the root of the tooth. Such an instrument can easily be used to place sponge as well as to remove periodontal structure bone regeneration composition after injecting of the composition and to place regeneration composition into wider pockets. The kit may also contain small dental burs 40 shown in Figure 8 that are used to abrade the inner gingival tissue and root surface. The dental burs of the prior art have a consistent diameter with a rounded or blunt end. Dental bur 40 has a specially shaped tip to allow easy penetration of the pocket, and markings such as lines 42 to indicate depth. The dental bur 40 has a smooth shank 41 and fluted tip 43 similar to 1156, 1157 and 1158 standard dental burs. The shank can be any desired length and diameter. One presently preferred tip comparable to the 1157 model is 0.39 inches (1.00 mm in diameter) and 0.165 inches (4.20 mm) long. The end of the tip 43 is blunt, but tapers to a rounded point. In a preferred embodiment there is 0.39 inches (1.0 mm) from the beginning of the taper to the end of the tip and the tip is rounded. The same specially shaped tip could have a 0.35 inch (0.9 mm) diameter comparable to an 1156, or have a 0.47 inch (1.2 mm) diameter comparable to an 1158. The kit may include more than one dental bur. If more than one size of bur is included, I prefer to color code the shaft of the burs to indicate size. The entire shaft could be colored or colored rings may be applied to the shaft.

It is further preferred to include a sponge counter in the kit. This can be a set of depressions 39 in the cover 29 of the kit 30. The depressions could be on an inside surface of the cover or on the outside surface as shown in Figure 6. Each kit will contain a known number of sponges. As the sponge is removed from the patient, one sponge is placed in each depression. Then the sponges are counted to assure that all those that were used have been removed from the patient.

It is further preferred to provide a dental bur brush 44 for cleaning the bur during the procedure. This brush preferably has two or three sets of opposed bristles 45 positioned so that the ends of the bristles will engage the dental bur as it is passed through the brush. The kit may also contain a tube of adhesive 46 for sealing the pocket after placement of the periodontal regeneration compound. A probe 48 having marks to measure pocket depth or an end configured to place sponge strips in the pocket may also be included.

The objective of the present method and composition is not merely to achieve bone growth, but rather to regenerate a healthy periodontal structure. This requires not only bone growth but also that the epithelial attachment regenerates. When the epithelial attachment reforms the lost bone beneath the seal will regenerate. Chewing imparts forces to the bone and the bone will grow and strengthen to match the forces.

### EXAMPLES OF PREPARATIONS OF PREFERRED COMPOSITIONS

I prefer to prepare the bone growth and adjacent tissue regeneration composition by mixing the bone growth material, free collagen, saline or other fluid and any desired additives, then heating the composition to cause the bone growth material to be captured between collagen fibers. A composition made in this way keeps the bone growth material from migrating and encourages tissue growth. Similar compositions can be made without heating, but those compositions are more likely to be displaced out of a periodontal pocket or move away from the bone growth site as a result of movement by adjacent tissue. The following examples illustrate present preferred compositions and methods of making same.

### Example 1

0.65 gram of hydroxyapatite was placed in a sealed nylon sleeve and heat sterilized for 1-2 hours at 350 degrees Fahrenheit. The hydroxyapatite and 0.5 grams of manufacturer-sterilized free collagen were placed in a 20ml covered plastic bottle and the ingredients mixed with a stainless steel spatula. To this mixture 0.6 ml of manufacturer-sterilized normal saline was added and the mixture was stirred again. The mixture was flattened out on the bottom of the bottle and the bottle was sealed and heated at 71 degrees Centigrade (160 degrees Fahrenheit) in a water bath for 2.5 to 5 minutes. After heating, the resulting paste was placed into a 1 cc plastic syringe. The loaded syringe can be held at a temperature of from 43°C to 71°C (110°F to 160°F) for immediate use or stored sealed in a 4 degrees Centigrade (39 degree Fahrenheit) refrigerator for up to four weeks. Additionally, the filled syringe can be frozen. When needed, the composition can be reheated in the syringe and then injected. This composition was stable for up to 36 hours in a 37 degrees Centigrade (98.6 degree Fahrenheit) water bath.

### Example 2

0.65 grams hydroxyapatite was sterilized as in Example 1. In a 20ml plastic bottle the hydroxyapatite and 0.12 grams free collagen were mixed together followed by the addition of 0.65 ml of saline and heated at 48 degrees Centigrade (120 degrees Fahrenheit) for 20 minutes. The paste had similar properties as that formed in Example 1.

### Example 3

0.65 grams hydroxyapatite was sterilized as in Example 1. In a 20ml plastic bottle the hydroxyapatite, 0.12 grams free collagen and 0.04 grams carageenam were mixed together followed by the addition of 0.65 ml of saline, mixing, flattening the mixture and then heating at 71 degrees Centigrade (160 degrees Fahrenheit) as in Example 1. The paste had similar properties as that formed in Example 1.

### Example 4

0.65 grams hydroxyapatite was sterilized as in Example 1. In a 20ml plastic bottle the hydroxyapatite, 0.16 grams collagen and 0.07 grams of ammonium chloride or calcium chloride were mixed together followed by the addition of 0.65 ml of saline, mixing, flattening the pellet and then heating at 71 degrees Centigrade (160 degrees Fahrenheit) as in Example 1. The paste had similar properties as that formed in Example 1.

### Example 5

0.65 grams hydroxyapatite was sterilized as in Example 1. In a 20ml plastic bottle the hydroxyapatite, 0.20 grams collagen were mixed together followed by the addition of 0.35 ml of saline and then 0.25 ml of vinegar, mixing, flattening the pellet and then heating at 71 degrees Centigrade (160 degrees Fahrenheit) as in Example 1. The paste had similar properties as that formed in Example 1.

### Example 6

0.65 grams hydroxyapatite was sterilized as in Example 1. In a 20ml plastic bottle the hydroxyapatite 0.50 grams collagen were mixed together followed by the addition of 0.65 ml of saline, mixed and then used as is. The resulting paste can then be used for the desired medical application, although slightly less viscous than the pastes in Examples 1-4.

### Example 7

0.65 grams hydroxyapatite was sterilized as in Example 1. In a 20ml plastic bottle the hydroxyapatite and 0.20 grams collagen were mixed together followed by the addition of 1.0 ml of saline and 0.065 grams doxycycline and heated at 71 degrees Centigrade (160 degrees Fahrenheit) as in Example 1. The paste had similar properties as that formed in Example 1.

### Example 8

0.65 grams hydroxyapatite was sterilized as in Example 1. In a 20ml plastic bottle the hydroxylapatite and 0.20 grams of collagen were mixed together followed by the addition of 0.70ml of saline and 0.13 grams of doxycycline and heated in a 2450 mHz microwave. The mixture was heated for 5 seconds followed by no heating for 5 sec. This heating/resting cycle continued for three cycles. The paste had similar properties as that formed in Example 1.

The composition was heated to cause the collagen fibers to dissociate but not degrade or denature proteins in the collagen. Dissociation has occurred when the collagen melts. A change in color indicates degradation. I heated several batches of the same composition at temperatures ranging from 43°C to 82°C (110°F to 180°F) in a water bath held at constant temperature to determine how long it took for the composition to melt and degrade at each temperature. The results of that test are set forth in Table 1. From these results it should be apparent that the composition must be heated 20 minutes at 43°C (110°F), but only 2 minutes at 82°C (180°F) to cause melting. Continued heating beyond 84 hours at 43°C (100°F) or beyond 20 hours at 82°C (180°F) caused degradation.

**TABLE 1**

| Temperature | Time to Melt | Time to Degrade |
|---|---|---|
| (110°F) 43°C | 20 minutes | 84 hours |
| (120°F) 48°C | 10 minutes | 84 hours |
| (130°F) 54°C | 7.5 minutes | 85 hours |
| (140°F) 60°C | 6.5 minutes | 66 hours |
| (150°F) 66°C | 5 minutes | 60 hours |
| (160°F) 71°C | 3 minutes | 58 hours |
| (170°F) 22°C | 2.5 minutes | 22 hours |
| (180°F) 82°C | 2 minutes | 20 hours |

While the times may differ depending upon the composition it is apparent from the data that compositions of the present invention can be maintained at an elevated temperature for several hours before use.

All of the materials used to formulate various embodiments of my bone growth composition have been routinely used for many years in the human body. Consequently, the formulations are safe and should meet applicable regulatory standards.

### EXAMPLES OF PATIENTS TESTED WITH BONE RE-GROWTH COMPOSITIONS

Various mixtures of hydroxyapatite with collagen have been tested in patients suffering periodontal disease. At no time on any patient reported herein have Guided Tissue Regeneration barriers been utilized. The bone regeneration with periodontal structure regeneration procedures alone has achieved similar or superior results as expected with Guided Tissue Regeneration barriers.

### Patient Example 1

This patient had generalized 6-10mm pockets on all 27 teeth. Without incisions, the bur of the type shown in Figure 8 was inserted between the tooth and gingiva completely to the bottom of the pocket. Using the dental drill at slow speed and with appropriate motion, all teeth were subjected to root and gingival abrasion by the bur until all root surfaces were clean and the inner lining of gingiva was abraded. The abraded root material was not rinsed out of the pocket. Sponge strips 5 mm, 7.5 mm and 10 mm wide were placed with the gauze placement instrument shown in Figure 7 into each pocket to create hemostasis and pocket distension. An original source of hydroxyapatite 300-400 micron particles was ground with a mortar and pestle until it was a fine microcrystalline hydroxyapatite powder capable of passing through an 18ga. needle. A preparation of 1:1 by volume of 1.0 mm sieved free collagen particles sold under the trademark Biocore® and microcrystalline hydroxyapatite mixed with sterile 0.9% saline to make the bone growth composition a paste consistency was loaded into a 1ml. Luer-Lok syringe. The sharp end of a standard 18ga. 1 ½ inch Luer-Lok needle was cut off and the end polishedto create the blunt needle through which the paste was injected. The sponge was removed after sufficient time elapsed for hemostasis and pocket distension. The periodontal structure regeneration material was then injected into the pocket between the tooth and gingiva until the pocket was filled. Butyl cyanoacrylate adhesive previously loaded into a micropipette was applied about 3mm on the tooth and 3mm on the gingiva at their junction to create a protective covering. The butyl cyanoacrylate covered all pockets filled with the bone growth composition. Water speeds the curing of butyl cyanoacrylate. Water was previously loaded into a micropipette and colored to distinguish the water filled pipette from the butyl cyanoacrylate filled pipette. Small droplets of water were placed on the butyl cyanoacrylate to speed curing. At the two-week post-surgery visit, the pockets were 2-5mm. At 8 weeks all pockets were 2-3mm. It was important that the adhesive filled pipette be readily distinguishable from the water filled pipette. Since butyl cyanoacrylate and water look alike one can easily pick up the wrong pipette. Adding a dye to one or the other permits easy distinction. However, since the adhesive remains in place it is better to color the water. One could also use differently shaped or marked pipettes.

### Patient Example 2

This patient required treatment of both mandibular teeth and maxillary teeth. The mandibular teeth were prepared and treated identically to the maxillary teeth. The only difference was the bone growth composition mixture used on the mandibular teeth. A mixture of 2:2:1 ofhydroxyapatite:free collagen:calcium sulfate by volume was placed into the pockets rather than the 1:1 hydroxyapatite:collagen used on the maxillary teeth. Despite the placement of the sponge, several sites continued to slightly ooze blood. Upon placement of the bone growth composition, bleeding ceased within 10-13 seconds. The surgery lasted 3 hours, no incisions were created, no overt bleeding occurred. Throughout the post-surgery period the patient did not require pain pills nor did he have swelling. At 8 week post-surgery, most pockets were 2-3mm with only 2 sites measuring 5mm. The gingiva remained at the same height with no recession and his oral cosmetics remained unchanged.

### Patient Example 3

This patient had 27 teeth with 5-9mm pockets and 11 furcation involvements. All teeth were prepared without incisions utilizing dental bur abrasion, sponge placement, material placement, and butyl cyanoacrylate covering most treated sites. A mixture of finely ground hydroxyapatite and free collagen particles at a 1:1 ratio by volume was injected into the prepared pockets until the pockets were filled. Some areas on the lingual of the mandible, and on the distal of terminal molars were impossible to cover with butyl cyanoacrylate. Those areas remained without a protective adhesive cover and healed as well as other butyl cyanoacrylate covered areas. At 9 weeks post-surgery, we were partially successful with 4 advanced furcation problems that remained with 5-7 mm pockets. All other pockets were 1-3 mm, with the exception of one site at 5mm. We were successful with 7 furcations that filled in with measurements of 2-3mm.

### Patient Example 4

This patient had teeth #21-27 remaining. These 7 teeth had extensive horizontal bone loss, but with much of the interproximal gingiva remaining. The 7-8mm pockets were located interproximal and were soft tissue in nature with little of the pocket resulting from osseous defects. The 7-8mm pockets occurred within 4 months. A particularly aggressive bacteria probably was the etiology of the rapid destruction of the periodontal structure called the epithelial attachment that attaches the gingiva to the tooth. To help destroy the bacteria, prior to placement, the sponge was soaked with an oxygenating solution. The solution releases oxygen. The oxygen causes both hemostasis and is bacteriocidal. All teeth were prepared without incisions utilizing dental bur abrasion, gauze placement, material placement, and butyl cyanoacrylate covering all treated sites. The material was 1:2 hydroxyapatite:free collagen by volume. By 4 weeks post-surgery, his pockets were all 2-3mm indicating that the periodontal structure regenerated.

### Patient Example 5

This patient had 27 teeth of which 25 were treated. Mobility on 17 of the teeth was from a minor + mobility to 1 on a standard dental scale. Pockets ranged from 6-8mm with 7 furcation problems. All teeth were prepared without incisions utilizing dental bur abrasion, sponge placement, material placement, and butyl cyanoacrylate covering all treated sites. Several persistent bleeding sites that would not stabilize with pressure, nor with injection of a Xylocaine with epinephrine, immediately ceased bleeding upon the placement of butyl cyanoacrylate over the sites. The bone growth composition was prepared with a ratio of 3:2:1 hydroxyapatite:free collagen:calcium sulfate. Included is a tally of the number of pocket readings pre-surgery and 12 week post-surgery on #4-13, 20-27. A total of 108 readings for the 18 teeth.

| Pre-surgery | Post-surgery |
|---|---|
| 1mm 0 | 1mm 66 |
| 2mm 13 | 2mm 28 |
| 3mm 36 | 3mm 14 |
| 4mm 26 | 4mm 0 |
| 5mm 14 | 5mm 0 |
| 6mm 8 | 6mm 0 |
| 7mm 10 | 7mm 0 |
| 8mm 1 | 8mm 0 |

The high number of post-surgery readings of 1mm and 2mm is extraordinary. Readings of 1mm are rarely seen in healthy patients, and few 2mm are seen in healthy patients. Mobility on all teeth was zero. One furcation completely healed, while 6 furcations reduced from 5-8mm to 2-5mm but were still Class I and Class II.

### Patient Example 6

Only 6 teeth were treated on a sixth patient, but the damage was severe. Teeth #9, 22-25, 27 are anterior teeth where cosmetics are paramount. Standard periodontal surgical treatment of this anterior area utilizing incisions removes large amounts of gingiva. Gingival recession with root exposure is a major problem for the periodontist, and of great cosmetic concern for the patient. Tooth #9 had an expensive ceramic facing adding an additional complication to treatment. Any gingival recession would expose the facing margin and defeat the cosmetic dentistry. All teeth were prepared without incisions utilizing dental bur abrasion, sponge placement, material placement, and butyl cyanoacrylate covering all treated sites. The material was 3:2:1 hydroxyapatite:free collagen:calcium sulfate. After 9 weeks, the pockets were all 1-2mm with one 3mm site. The 10mm site was now 2mm. No gingival recession occurred. There were no cosmetic complications and the ceramic facing was not exposed.

### Patient Example 7

This patient had 6 to 10 mm pockets. Fourteen days after treatment the pockets were about 4 mm deep. This decreased depth cannot be from bone regeneration in such a short time. Bone really requires many weeks and months to fully form. Bone precursor tissue grew and filled in the space, but that still does not account for the rapid decrease in pocket depth. The soft tissue structures of gingiva, underlying fibrous connective tissue, periodontal ligament, and epithelial attachment probably were forming concurrently thus decreasing the depth. Much of the greatly enhanced speed of healing comes from the properties attributed to free collagen. Before healing can begin, hemostasis must occur. Medical literature states that collagen applied to a wound at the time of hemostasis acts as an assistive mechanism to augment clotting. Collagen actually increases platelet adherence to the endothelial vessel walls, thus sealing them off. Fibroblasts combine and build amino acids into a sugar protein structure to form collagen at the wound site. This advances the wound environment by five or six days. Collagen also attracts additional monocytes to the wound, thus increasing the amount of debris removed, which leads to speedier wound healing. During angiogenesis, collagen assists with epithelialization of the endothelial lining of the blood vessel wall. By developing this system a healthy wound will develop and healing occurs.

The particular compositions used to treat these seven patients contained collagen material that I obtained from BioCore, Inc. I also treated patients using a composition that contained hydroxylapatite and collagen obtained from other sources. One such collagen was Avitene collagen sold in sheets 35 mm x 35 mm x 1 mm. The sheets crumbled under very light pressure. I mixed the Avitene collagen with the hydroxylapatite powder and saline and applied them to the patient. The Avitene collagen did not affect the rate of healing. I had obtained similar results using hydroxyapatite without collagen.

I also treated a patient with a mixture of hydroxyapatite and Collastat collagen. The Collastat collagen is a fibrous material similar to a tuft of cotton. I was unable to cut the Collastat collagen into pieces small enough to pass through an 18 gauge needle. Therefore, I applied the collagen and hydroxyapatite using spatulas to periodontal pockets. The results using the Collastat collagen were no different than results achieved without collagen.

I attempted to determine why the BioCore collagen speeded healing and bone regeneration while the Avatene collagen and the Collastat collagen did not. BioCore would not reveal the composition or method of manufacture of BioCore collagen. However, I did learn that unlike other collagens available in the market, BioCore collagen is not treated with glutaraldehyde. Glutaraldehyde acts as a cross-linker of collagen molecules. BioCore stated that their collagen is the only collagen that is not hydrolyzed or further cross-linked, worldwide. Furthermore, 90% of the fibrillar collagen is still intact after their mild proprietary processing methods.

I believe that the Avitene collagen and the Collastat collagen are cross-linked structures. However, they did not speed healing like the BioCore collagen. Apparently, the cross-linking prevents the Avitene and Collastat collagen from acting like the free collagen. Jeffries in United States Patent Nos. 4,394,370 and 4,472,840 describes collagen treated with glutaraldehyde as forming a sponge when dried. Chen et al. United States Patent No. 5,707,962 discloses methods of using this collagen sponge described by Jeffries. Consequently, the composition disclosed by Jeffries and Chen, in which collagen has been cross-linked, would not be expectedto speed healing, like the compositions disclosed here containing free collagen.

From the foregoing examples it can be seen that the periodontal structure regeneration material can vary in composition but contains at least one bone growth material and free collagen. Suitable materials include a mixture of a fine microcrystalline hydroxylapatite powder, some 1 to 400 micron hydroxylapatite particles, free collagen particles, and calcium sulfate in varying percentages. This collagen has been used in small particles less than 1 mm, and collagen has been used in a gel form in the mixtures. The hydroxylapatite used in the mixture was 100% microcrystalline, or a mixture of mostly microcrystalline material with some larger particles varying from approximately 1 micron to about 400 microns in diameter. Calcium sulfate has been used in varying percentages. I have also used finely ground bone with collagen in varying percentages. Other periodontal regeneration materials contained bovine bone used as a finely ground powder and free collagen in varying percentages. Various ceramic glass bone growth materials have been used with free collagen in varying percentages. All of these variations have created periodontal structure regeneration.

Although I have disclosed several formulations in which BioCore collagen was used, the invention is not limited to compositions containing collagen provided by BioCore, Inc. Any collagen material can be used that contains collagen particles that are not cross-linked and that speeds healing. The composition may also include other materials in addition to those disclosed here, provided those other materials do not cause significant amounts of free collagen to crosslink and those other materials are suitable for use in a periodontal pocket or other site of bone loss.

## Claims

1. A bone growth and adjacent tissue regeneration composition for treatment of bone loss comprising a mixture of particles of a bone growth material, collagen and a fluid, **characterized in that** the collagen is present in the form of free collagen fibres, being collagen in a form which is not cross-linked.

2. The composition of claim 1, also comprising a thickener.

3. The composition of claim 2 wherein the thickener is a physiologically compatible compound which is liquid above physiological temperature.

4. The composition of claim 3 wherein the compound is an oligosaccharide.

5. The composition of claim 4 wherein the oligosaccharide is a polyethylene glycol.

6. The composition of claim 2 wherein the thickener is a material that is activated by at least one of air, heat, light and a catalyst.

7. The composition of claim 2 wherein the thickener is a material that absorbs water.

8. The composition of claim 7 wherein the thickener is a hydrogel or corn starch.

9. The composition of claim 2 in which the thickener added to the mixture is a physiological compatible compound that slowly forms an insoluble salt with at least one component in the mixture.

10. The composition of any preceding claim, further comprising an adhesive placed over a portion of the mixture, the adhesive being selected from the group comprising water curable adhesives, visible light curable adhesives, auto curing adhesives, heat curable adhesives, reactive-component cure adhesives, surface activated adhesives and curable adhesives that cure when exposed to ultraviolet radiation.

11. The composition of any preceding claim, wherein the mixture is subjected to infusion of energy from at least one energy source sufficient to dissociate the collagen fiber without denaturing proteins in the collagen fibers and to allow at least some of the particles of bone growth material to be captured between adjacent collagen fibers after the infusion of energy has ceased.

12. The composition of claim 11 in which the energy source is a water bath, an oil bath, a microwave source, a radiant heat source or a sonicator.

13. The composition of claim 10 wherein the infusion of energy is provided by heating the composition in one of an oven, a water bath and an oil bath.

14. The composition of any of claims 1 to 10, wherein the composition is heated to a temperature at least 43° (110°F) for a time period sufficient to cause the collagen fiber in the composition to melt but not to degrade.

15. The composition of claim 14 in which the heat is provided by a water bath between 43°C and 82°C (110 and 180 degrees Fahrenheit).

16. The composition of claim 14 in which the heat is provided by a water bath between 48°C and 71°C (120 and 160 degrees Fahrenheit) for less than 20 minutes.

17. The composition of any preceding claim, further comprising at least one material selected from the group consisting of growth factors, nutrient factors, drugs, calcium containing compounds, anti-inflammatory agents, anti-microbial agents, root treatment materials, bone morphogenic proteins, dental matrix derivatives and combinations thereof.

18. The composition of any preceding claim, wherein the bone growth material is a material selected from the group consisting of human bone particles, hydroxyapatite, animal bone particles, ground coral, calcium sulfate, calcium carbonate, substituted and unsubstituted calcium phosphate cements, bioactive glasses, polymers that promote bone growth and combinations thereof.

19. The composition of claim 18 in which the calcium phosphate cements are chosen from synthetic or commercially available hydroxyapatite and substituted hydroxyapatites.

20. The composition of claim 19 in which at least a portion of at least one of the phosphate, calcium and hydroxide components of the calcium phosphate cements is substituted by simple inorganic ions (one element type) and/or complex inorganic ions (having more than one element type).

21. The composition of any preceding claim, also comprising at least one matrix forming material selected from the group consisting of cross-linked collagen, water soluble polymers, gelling polymers, hydrocolloids, hydrogels, polyethyleneglycols, natural polymers, chemically modified natural polymers, synthetic polymers and copolymers, colloids, thermosetting polymers and thermoplastic polymers.

22. The composition of claim 21 in which at least one of the matrix-forming materials gets more viscous at or below 37°C (98.6 degrees Fahrenheit).

23. The composition of claim 21 in which at least one of the matrix-forming materials melts at or above 37°C (98.6 degrees Fahrenheit).

24. The composition of any preceding claim, in which the composition, after formulation, is stored in either a refrigerator at a temperature above 0°C (32°F) and a freezer at a temperature below 0°C (32°F).

25. The composition of any preceding claim, in which the fluid is selected from the group of biocompatible fluids consisting of normal saline, water and inorganic salt solutions.

26. The composition of any preceding claim, in which the fluid is comprised of a biocompatible, water-miscible organic solvent up to 10% of total fluid volume.

27. The composition of any preceding claim, wherein the growth material is anhydrous calcium sulfate.

## Patentansprüche

1. Eine Knochenwachstums- und benachbartes Gewebe regenerierende Zusammensetzung für die Behandlung von Knochenschwund, bestehend aus einer Mischung von Partikeln eines Knochenwachstumsmaterials, Kollagen und einer Flüssigkeit, **dadurch gekennzeichnet, dass** das Kollagen in der Form von freien Kollagenfasern vorhanden ist, nämlich als ein Kollagen in einer Form, die nicht quervernetzt ist.

2. Die Zusammensetzung nach Anspruch 1, weiter einen Eindicker beinhaltend.

3. Die Zusammensetzung nach Anspruch 2, wobei der Eindicker eine physiologisch kompatible Verbindung ist, welche über einer physiologischen Temperatur flüssig ist.

4. Die Zusammensetzung nach Anspruch 3, wobei die Verbindung ein Oligosaccharid ist.

5. Die Zusammensetzung nach Anspruch 4, wobei das Oligosaccharid ein Polyethylenglykol ist.

6. Die Zusammensetzung nach Anspruch 2, wobei der Eindicker ein Material ist, welches durch wenigstens einen von Luft, Wärme, Licht und einen Beschleuniger aktiviert wird.

7. Die Zusammensetzung nach Anspruch 2, wobei der Eindicker ein Material ist, welches Wasser absorbiert.

8. Die Zusammensetzung nach Anspruch 7, wobei der Eindicker ein Hydrogel oder Maisstärke ist.

9. Die Zusammensetzung nach Anspruch 2, in welcher der zu der Mischung hinzugefügte Eindicker eine physiologisch kompatible Verbindung ist, welche langsam ein nicht lösliches Salz mit wenigstens einer Komponente in der Mischung formt.

10. Die Zusammensetzung nach einem vorhergehenden Anspruch, weiter ein über einen Teil der Mischung platziertes Haftmittel beinhaltend, dasselbe Haftmittel aus der Gruppe bestehend aus wasserhärtenden Haftmitteln, in sichtbarem Licht härtenden Haftmitteln, automatisch härtenden Haftmitteln, wärmehärtenden Haftmitteln, durch reaktive Verbindung härtenden Haftmitteln, tensidaktivierten Haftmitteln und aushärtbaren Haftmitteln ausgewählt, welche aushärten wenn sie ultravioletter Bestrahlung ausgesetzt werden.

11. Die Zusammensetzung nach einem vorhergehenden Anspruch, wobei die Mischung der Infusion von Energie aus wenigstens einer Energiequelle ausgesetzt wird, welche ausreicht um die Kollagenfasern zu dissoziieren ohne Proteine in den Kollagenfasern zu vergällen, und das Festhalten von wenigstens einigen der Partikel des Knochenwachstumsmaterials zwischen nebeneinander liegenden Kollagenfasern nach Beenden der Infusion von Energie erlaubt.

12. Die Zusammensetzung nach Anspruch 11, in welcher die Energiequelle ein Wasserbad, ein Ölbad, eine Mikrowellenquelle, eine Strahlungswärmequelle oder ein Sonicator ist.

13. Die Zusammensetzung nach Anspruch 10, wobei die Infusion von Energie durch Erwärmen der Zusammensetzung in einem Ofen, einem Wasserbad und einem Ölbad erzeugt wird.

14. Die Zusammensetzung nach einem der Ansprüche 1 bis 10, wobei the Zusammensetzung für eine für das Schmelzen der Kollagenfasern in der Zusammensetzung ausreichende, aber diese nicht abbauende, Zeitperiode auf eine Temperatur von mindestens 43° (110°F) erwärmt wird.

15. Die Zusammensetzung nach Anspruch 14, in welcher die Wärme durch ein Wasserbad zwischen 43°C and 82°C (110 und 180 Grad Fahrenheit) erzeugt wird.

16. Die Zusammensetzung nach Anspruch 14, in welcher die Wärme durch ein Wasserbad zwischen 48°C und 71°C (120 und 160 Grad Fahrenheit) erzeugt wird.

17. Die Zusammensetzung nach einem vorhergehenden Anspruch, weiter wenigstens ein Material, ausgewählt aus der aus Wachstumsfaktoren, Nährstofffaktoren, Medikamenten, kalziumhaltigen Verbindungen, Entzündungshemmern, antimikrobiellen Mitteln, Wurzelbehandlungsmaterialen, knochenmorphogenetischen Proteinen, Zahnmatrixderivativen und Kombinationen derselben bestehenden Gruppe beinhaltend.

18. Die Zusammensetzung nach einem vorhergehenden Anspruch, wobei das Knochenwachstumsmaterial ein aus einer menschliche Knochenpartikel, Hydroxyapatit, tierische Knochenpartikel, gemahlene Koralle, Kalziumsulfat, Kalziumkarbonat, substituierte und nicht substituierte Kalziumphosphatzemente, bioaktives Glas, den Knochenwachstum fördernde Polymer und Kombinationen derselben beinhaltenden Gruppe ausgewähltes Material ist.

19. Die Zusammensetzung nach Anspruch 18, in welcher die Kalziumphosphatzemente aus synthetischen oder kommerziell vertriebenen Hydroxyapatiten und substituierten Hydroxyapatiten ausgewählt werden.

20. Die Zusammensetzung nach Anspruch 19, in welcher wenigstens ein Anteil von mindestens einer der Phosphat-, Kalzium- und Hydroxidkomponenten der Kalziumphosphatzemente durch einfache anorganische Ionen (ein Elementtyp) und/oder komplexe anorganische Ionen (mit mehr als einem Elementypen) substituiert wird.

21. Die Zusammensetzung nach einem vorhergehenden Anspruch, weiter wenigstens ein aus der Gruppe von quervernetztem Kollagen, wasserlöslichen Polymern, gellierenden Polymern, Hydrokolloiden, Hydrogels, Polyethylenglykolen, natürlichen Polymern, chemisch abgeänderten natürlichen Polymer, synthetischen Polymern und Co-Polymern, Kolloiden, wärmehärtenden Polymern und duroplastischen Polymern bestehendes ausgewähltes Matrix-formendes Material beinhaltend.

22. Die Zusammensetzung nach Anspruch 21, in welcher wenigstens eines der Matrixformenden Materiale bei oder unter 37°C (98,6 Grad Fahrenheit) zähflüssiger wird.

23. Die Zusammensetzung nach Anspruch 21, in welcher wenigstens eines der Matrixformenden Materiale bei oder über 37°C (98,6 Grad Fahrenheit) schmilzt.

24. Die Zusammensetzung nach einem vorhergehenden Anspruch, in welcher die Zusammensetzung nach ihrer Formulierung entweder in einem Kühlschrank bei einer Temperatur von über 0°C (32°F) oder in einem Gefrierschrank bei einer Temperatur von über 0°C (32°F) aufbewahrt wird.

25. Die Zusammensetzung nach einem vorhergehenden Anspruch, in welcher die Flüssigkeit aus der biokompatible Flüssigkeiten wie normale salzhaltige, Wasser und anorganische Salzlösungen beinhaltenden Gruppe ausgewählt wird.

26. Die Zusammensetzung nach einem vorhergehenden Anspruch, in welcher die Flüssigkeit bis zu 10% des Gesamtflüssigkeitsvolumens ein biokompatibles, wassermischbares organisches Lösungsmittel beinhaltet.

27. Die Zusammensetzung nach einem vorhergehenden Anspruch, wobei das Wuchsmaterial ein wasserfreies Kalziumsulfat ist.

## Revendications

1. Composition pour la croissance osseuse et la régénération des tissus adjacents pour le traitement de la perte osseuse, comprenant un mélange de particules d'une matière de croissance osseuse, un collagène et un fluide, **caractérisée en ce que** le collagène est présent sous la forme de fibres de collagène libres, le collagène étant sous une forme qui n'est pas réticulée.

2. Composition selon la revendication 1, comprenant en outre un épaississant.

3. Composition selon la revendication 2, dans laquelle l'épaississant est un composé physiologiquement compatible qui est liquide au-dessus de la température physiologique.

4. Composition selon la revendication 3, dans laquelle le composé est un oligosaccharide.

5. Composition selon la revendication 4, dans laquelle l'oligosaccharide est un polyéthylène glycol.

6. Composition selon la revendication 2, dans laquelle l'épaississant est une matière qui est activée par l'un au moins de l'air, la chaleur, la lumière et un catalyseur.

7. Composition selon la revendication 2, dans laquelle l'épaississant est une matière qui absorbe l'eau.

8. Composition selon la revendication 7, dans laquelle l'épaississant est un hydrogel ou de l'amidon de maïs.

9. Composition selon la revendication 2, dans laquelle l'épaississant ajouté au mélange est un composé physiologique compatible qui forme lentement un sel insoluble avec au moins un composant dans le mélange.

10. Composition selon l'une quelconque des revendications précédentes, comprenant en outre un adhésif placé sur une partie du mélange, l'adhésif étant choisi parmi le groupe comprenant des adhésifs durcissables à l'eau, des adhésifs durcissables à la lumière visible, des adhésifs auto-durcissants, des adhésifs thermo-durcissables, des adhésifs durcis par un composant réactif, des adhésifs activés en surface et des adhésifs durcissables qui durcissent lorsqu'ils sont exposés à un rayonnement ultraviolet.

11. Composition selon l'une quelconque des revendications précédentes, dans laquelle le mélange est soumis à l'infusion d'énergie à partir d'au moins une source d'énergie suffisante pour dissocier la fibre de collagène sans dénaturer les protéines dans les fibres de collagène et pour permettre de capturer au moins certaines particules de matière de croissance osseuse entre des fibres de collagène adjacentes après que l'infusion d'énergie a cessé.

12. Composition selon la revendication 11, dans laquelle la source d'énergie est un bain d'eau, un bain d'huile, une source de micro-ondes, une source de chaleur rayonnante ou un émetteur d'ultrasons.

13. Composition selon la revendication 10, dans laquelle l'infusion d'énergie est assurée en chauffant la composition dans l'un parmi une étuve, un bain d'eau et un bain d'huile.

14. Composition selon l'une quelconque des revendications 1 à 10, la composition étant chauffée jusqu'à une température d'au moins 43°C (110°F) pendant une période de temps suffisante pour amener la fibre de collagène dans la composition à fondre mais sans se dégrader.

15. Composition selon la revendication 14, dans laquelle la chaleur est fournie par un bain d'eau entre 43°C et 82°C (100 et 180 degrés Fahrenheit).

16. Composition selon la revendication 14, dans laquelle la chaleur est fournie par un bain d'eau entre 48°C et 71°C (120 et 160 degrés Fahrenheit) pendant moins de 20 minutes.

17. Composition selon l'une quelconque des revendications précédentes, comprenant en outre au moins une matière choisie parmi le groupe constitué de facteurs de croissance, de facteurs nutritifs, de médicaments, de composés contenant du calcium, d'agents anti-inflammatoires, d'agents antimicrobiens, de matières de traitement de la racine, de protéines morphogéniques osseuses, de dérivés de matrice dentaire et de combinaisons de ceux-ci.

18. Composition selon l'une quelconque des revendications précédentes, dans laquelle la matière de croissance osseuse est une matière choisie parmi le groupe constitué de particules osseuses humaines, d'hydroxyapatite, de particules osseuses animales, de corail broyé, de sulfate de calcium, de carbonate de calcium, de ciments de phosphate de calcium substitués et non substitués, de verres bioactifs, de polymères qui favorisent la croissance osseuse et de combinaisons de ceux-ci.

19. Composition selon la revendication 18, dans laquelle les ciments de phosphate de calcium sont choisis parmi une hydroxyapatite synthétique ou disponible dans le commerce et des hydroxyapatites substituées.

20. Composition selon la revendication 19, dans laquelle au moins une partie d'au moins un des composants phosphate, calcium et hydroxyde du ciment de phosphate de calcium est remplacée par des ions inorganiques simples (un seul type d'élément) et/ou des ions inorganiques complexes (ayant plus d'un type d'élément).

21. Composition selon l'une quelconque des revendications précédentes, comprenant en outre au moins une matrice formant une matière choisie parmi le groupe constitué de collagène réticulé, de polymères solubles dans l'eau, de polymères gélifiants, d'hydrocolloïdes, d'hydrogels, de polyéthylèneglycols, de polymères naturels, de polymères naturels chimiquement modifiés, de polymères et de copolymères synthétiques, de colloïdes, de polymères thermodurcissables et de polymères thermoplastiques.

22. Composition selon la revendication 21, dans laquelle au moins une des matières formant la matrice devient plus visqueuse à ou en dessous de 37°C (98,6 degrés Fahrenheit).

23. Composition selon la revendication 21, dans laquelle au moins une des matières formant la matrice fond à ou au-dessus de 37°C (98,6 degrés Fahrenheit).

24. Composition selon l'une quelconque des revendications précédentes, la composition, après formulation, étant stockée soit dans un réfrigérateur à une température au-dessus de 0°C (32°F) soit dans un congélateur à une température en dessous de 0°C (32°F).

25. Composition selon l'une quelconque des revendications précédentes, dans laquelle le fluide est choisi parmi le groupe de fluides biocompatibles constitué de solution physiologique salée, d'eau et de solutions de sels inorganiques.

26. Composition selon l'une quelconque des revendications précédentes, dans laquelle le fluide est constitué d'un solvant organique biocompatible, miscible à l'eau jusqu'à 10 % du volume total de fluide.

27. Composition selon l'une quelconque des revendications précédentes, dans laquelle la matière de croissance est du sulfate de calcium anhydre.
